(19) Europäisches Patentamt
European Patent Office
Office européen des brevets

(11) Veröffentlichungsnummer: **0 285 755**
**A2**

# (12) EUROPÄISCHE PATENTANMELDUNG

(21) Anmeldenummer: **88101430.2**

(22) Anmeldetag: **02.02.88**

(51) Int. Cl.⁴: **C07C 33/044 , C07C 29/42**

(30) Priorität: **04.04.87 DE 3711382**

(43) Veröffentlichungstag der Anmeldung:
**12.10.88 Patentblatt 88/41**

(84) Benannte Vertragsstaaten:
**BE DE FR GB IT NL**

(71) Anmelder: **HÜLS AKTIENGESELLSCHAFT**
**Patentabteilung / PB 15 - Postfach 13 20**
**D-4370 Marl 1(DE)**

(72) Erfinder: **Thelen, Gerhard, Dr.**
**Sepp-Herberger-Strasse 62**
**D-4405 Nottuln(DE)**

## (54) Verfahren zur Herstellung von Alkindiolen durch Umsetzung von Ketonen mit Acetylen.

(57) Alkindiole werden durch Umsetzung von Ketonen mit Acetylen in Gegenwart einer Base erhalten, bisher in organischen Lösemitteln wie Acetaldehyd-dibutylacetal, Methylal, Dioxan, Diisopropylether, Tetrahydrofuran oder Kohlenwasserstoffen. Meist sind diese Suspensionen sehr viskos. Zudem ist der Umsatz des Ketons im allgemeinen unvollständig.

Durch Verwendung von Alkyl-tert.-butylether, vorzugsweise Methyl-tert.-butylether, als Lösemittel beseitigt man die Nachteile und erhält bei guten Umsätzen hohe Ausbeuten.

Herstellung von 2.4.7.9-Tetramethyldec-5-in-4.7-diol, 2.5.8.11-Tetramethyldodec-6-in-5,8-diol

EP 0 285 755 A2

## Verfahren zur Herstellung von Alkindiolen durch Umsetzung von Ketonen mit Acetylen

Für die Herstellung der Alkindiolen der allgemeinen Formel I

$$R_1 - \underset{OH}{\overset{R_2}{C}} - C \equiv C - \underset{OH}{\overset{R_3}{C}} - R_4$$

sind eine Reihe von Herstellungsmethoden beschrieben. Im Falle der Aldehyde lassen sich Formaldehyd und Acetaldehyd nach dem Reppeschen Ethinylierungsverfahren mit Kupferacetylid als Katalysator sehr gut mit Acetylen zu den entsprechenden Monoalkoholen und Glykolen umsetzen. Bei höheren Aldehyden führt diese Methode zu unbefriedigenden Ergebnissen.

Besondere Schwierigkeiten bereitet die Umsetzung von zwei Mol eines Ketons mit einem Mol Acetylen. Hierbei ist mindestens ein Mol einer Base erforderlich. Die bisher beschriebenen Verfahren verwendeten feinverteiltes, wasserfreis Kaliumhydroxidpulver in organischen Lösemitteln wie Acetaldehyddibutylacetal (US-PSS 2 385 546, 2 385 548 und 2 455 058), Methylal und Dioxan (siehe W. Ziegenbein: Einführung der Äthinyl-und Alkinyl-Gruppe in organische Verbindungen, Verlag Chemie (1963), Diisopropylether (US-PS 2 163 720) oder Tetrahydrofuran (DE-AS 26 28 145). Ein höherer Wassergehalt des Kaliumhydroxids bewirkt einen steigenden KOH-Verbrauch.

Meist sind solche Suspensionen in den oben genannten Lösemitteln so viskos, daß ein gutes Durchmischen erheblich erschwert ist. Ein größerer Überschuß an Lösemittel ist oft nicht wirtschaftlich, da die bisher eingesetzten Lösemittel recht teuer sind. Ferner erfordert deren Wiedergewinnung zum erneuten Einsatz kostspielige Maßnahmen.

Das in den DE-PSS 20 08 675 und 20 47 446 beschriebene Verfahren setzt Kaliumalkoholate aliphatischer Alkohole in leicht zugänglichen aliphatischen, cycloaliphatischen oder aromatischen Kohlenwasserstoffen als Lösemittel ein. Allerdings ist die Tatsache, daß in einer vorgelagerten Reaktion die Suspension aus feinverteiltem Kaliumalkoholat im Lösemittel hergestellt werden muß, von Nachteil.

Bei der Umsetzung längerkettiger, aliphatischer Methylketone wird z. B., wie in der DE-PS 26 28 145 beschrieben, in Tetrahydrofuran unter Verwendung von technischem Kaliumhydroxid und trotz eines Verhältnisses KOH zu Keton von 2 : 1 nur ein unvollständiger Umsatz des Ketons erreicht. Zusätzlich fällt meist ein Gemisch des Monoalkohols und des gewünschten Acetylenglykols der allgemeinen Formel I an.

Daraus ergab sich die Aufgabe, ein Verfahren zu finden, das die Herstellung von Alkindiolen durch Umsetzung von Ketonen mit Acetylen bei weitgehendem Umsatz und hohen Ausbeuten in wirtschaftlicher Weise ermöglicht.

Diese Aufgabe wird erfindungsgemäß entsprechend den Angaben der Patentansprüche gelöst.

Überraschenderweise wurde nun gefunden, daß bei Verwendung von Alkyl-tert.-butylether, vorzugsweise Methyl-tert.-butylether (MTB) und/oder Ethyl-tert.-butylether (ETB), insbesondere Methyl-tert.-butylether, als Lösemittel die oben erwähnten Nachteile nicht auftreten. Als basisches Mittel setzt man ein Kaliumhydroxid-Pulver ein, bevorzugt mit einem Wassergehalt von 10 bis 24 %, insbesondere von 12 bis 15 %. Dabei führt man die Umsetzung der Carbonylverbindungen mit Acetylen zu den entsprechenden Alkindiolen zweckmäßigerweise bei Temperaturen von 20 bis 55 °C, bevorzugt von 30 bis 50 °C, durch, in dem vorzugsweise die Carbonylkomponente und das Acetylen in einem der angestrebten Reaktion entsprechenden stöchiometrischen Verhältnis synchron in die Suspension, bestehend aus Kaliumhydroxid und Alkyl-tert.-butylether, eingeleitet werden. Man erhält gut rührbare Reaktionsgemische. Vorzugsweise setzt man das Keton und das Acetylen im Molverhältnis von 1 bis 3 : 1 ein, insbesondere von 1,8 bis 2,2 : 1.

Nach beendeter Reaktion versetzt man mit Wasser und trennt durch Phasentrennung KOH nahezu quantitativ als Kalilauge ab. Die organische Phase wird mit einer organischen Säure, z. B. Ameisensäure, Essigsäure oder Homologe, neutralisiert und destillativ aufgetrennt. Das auf diese Weise zurückgewonnene Lösemittel ist ohne weitere Reinigungsschritte erneut verwendbar.

Man arbeitet im allgemeinen unter Atmosphärendruck.

Als Carbonylverbindungen kommen aliphatische, araliphatiche, aromatische und cyclische Ketone in Betracht, insbesondere sind Aceton, Methylethylketon, Methylisobutylketon, Methylisoamylketon und Cyclohexanon geeignet.

Man führt das Verfahren erfindungsgemäß durch, indem man die Komponenten Kaliumhydroxid und Keton in einem Molverhältnis von 1,0 bis 1,6 : 1, insbesondere von 1,2 bis 1,5 : 1, zur Reaktion bringt.

Die nach diesem Verfahren gewonnenen Produkte sind wertvolle Zwischenprodukte für die Pharma-und Riechstoffindustrie. Andere Acetylenglykole werden zur Herstellung oberflächenaktiver Formulierungen verwendet. Durch Umsetzung mit

Wasserstoffperoxid entstehen Hydroperoxide, die als Polymerisationsinitiatoren eingesetzt werden.

Beispiel 1

In einem mit Rührer versehenen Reaktor werden in 600 ml Methyl-tert.-butylether 478 g Kaliumhydroxid-Pulver (88prozentig, Rest $H_2O$) suspendiert und gleichzeitig 5 mol Methylisobutylketon (MIBK) und 60 l Acetylen eingeleitet. Durch Kühlung wird die Temperatur bei 35 °C konstant gehalten. Nach beendeter Umsetzung isoliert man nach Hydrolyse mit 3,4 l Wasser aus der organischen Phase durch Vakuumdestillation 458 g 2.4.7.9.-Tetramethyldec-5-in-4.7-diol. Dies entspricht einer Ausbeute von 81 %, bezogen auf die eingesetzte MIBK-Menge. Der Anteil des Monoalkohols ist mit 5,5 % gering.

Vergleichbare Ergebnisse erhält man, wenn man anstelle von Methyl-tert.-butylether den Ethyl-tert.-butylether einsetzt.

Beispiel 2

Führt man die Umsetzung wie in Beispiel 1 beschrieben mit 542 g Kaliumhydroxid-Pulver (88prozentig) und 5,7 mol MIBK sowie 68 l Acetylen durch, so lassen sich 79 % des eingesetzten Ketons in Form des Acetylenglykols neben 7,5 % des Monoalkohols isolieren.

Beispiel 3

Analog zu der Verfahrensweise nach Beispiel 1 setzt sich MIBK bei einer konstanten Reaktionstemperatur von 40 °C unter Verwendung von wassergesättigtem MTB als Lösemittel zu 78 % zum Acetylenglykol und zu 3,4 % zum Monoalkohol um.

Beispiel 4

Nach Beispiel 1 werden bei einer konstanten Reaktionstemperatur von 40 °C aus 6 mol MIBK und 72 l Acetylen 448 g 2.4.7.9-Tetramethyl-dec-5-in-4.7-diol erhalten. Aus 9 % der Ketoneinsatzmenge bildet sich der Monoalkohol.

Beispiel 5

Wie in Beispiel 3 beschrieben entstehen bei einer Reaktionstemperatur von 40 °C aus 3 mol Methylisoamylketon und 36 l Acetylen in Gegenwart von 287 g Kaliumhydroxid-Pulver 229 g 2.5.8.11-Tetramethyldodec-6-in-5.8-diol. Der Umsatz zum Monoalkohol ist mit 5 % gering.

Vergleichsbeispiel A

In eine gut rührbare Suspension aus 600 ml wasserfreiem Tetrahydrofuran und 478 g Kaliumhydroxid-Pulver (88prozentig) werden 5 mol MIBK und 60 l Acetylen bei konstant gehaltener Temperatur von 35 °C umgesetzt. Nach Hydrolyse und Phasentrennung lassen sich durch Vakuumdestillation 400 g des Acetylenglykols (entspricht 71 % des eingesetzten Ketons) sowie 10 % in Form des Monoalkohols gewinnen.

**Ansprüche**

1. Verfahren zur Herstellung von Alkindiolen durch Umsetzung von Ketonen mit Acetylen in Gegenwart von Kaliumhydroxid,
dadurch gekennzeichnet,
daß man die Umsetzung in Alkyl-tert.-butylether als Lösemittel durchführt und das Kaliumhydroxid in einem Molverhältnis zum Keton von 1,0 bis 1,6 : 1 einsetzt.

2. Verfahren nach Anspruch 1,
dadurch gekennzeichnet,
daß man als Alkyl-tert.-butylether den Methyl-tert.-butylether einsetzt.

3. Verfahren nach den Ansprüchen 1 und 2,
dadurch gekennzeichnet,
daß man das Kaliumhydroxid in einem Molverhältnis zum Keton von 1,2 bis 1,5 : 1 einsetzt.

4. Verfahren nach einem der Ansprüche 1 bis 3,
dadurch gekennzeichnet,
daß man als Keton Aceton, Methylethylketon, Methylisobutylketon, Methylisoamylketon und Cyclohexanon einsetzt.

5. Verfahren nach einem der Ansprüche 1 bis 4,
dadurch gekennzeichnet,
daß man das Keton und das Acetylen im Molverhältnis von 1 bis 3 : 1, vorzugsweise von 1,8 bis 2,2 : 1 einsetzt.

6. Verfahren nach einem der Ansprüche 1 bis 5,
dadurch gekennzeichnet,

daß man ein Kaliumhydroxid-Pulver mit einem Wassergehalt von 10 bis 24 %, vorzugsweise von 12 bis 15 %, eingesetzt.